# EUROPEAN PATENT SPECIFICATION

(11) **EP 1 605 035 B1**
(45) Date of publication and mention of the grant of the patent: **19.09.2007**
(21) Application number: 05103883.4
(22) Date of filing: 10.05.2005
(51) Int. Cl.: C11B 9/00, C07C 35/36, C07C 35/37, C07C 49/447, C07C 49/637, C07C 49/643, C07C 69/013

(54) **Perfuming ingredients of the woody type**
Parfümierende Bestandteile des holzigen Typs
Ingrédients parfumants du type boisé

(30) Priority: 08.06.2004 EP 04102577
(43) Date of publication of application: 14.12.2005
(73) Proprietor: FIRMENICH SA, 1211 Genève 8 (CH)
(72) Inventor: Moretti, Robert, 1205, GENEVA (CH); Etter, Olivier, 1225, CHENE-BOURG (CH)
(74) Representative: Schneiter, Sorin

(56) References cited:
- US-A- 4 118 343
- US-A- 4 311 852
- US-A- 4 377 714
- US-A- 4 671 798
- US-A- 5 114 915

## Description

### Technical field

The present invention relates to the field of perfumery. More particularly, it concerns the use as perfuming ingredient of a decaline derivative of formula 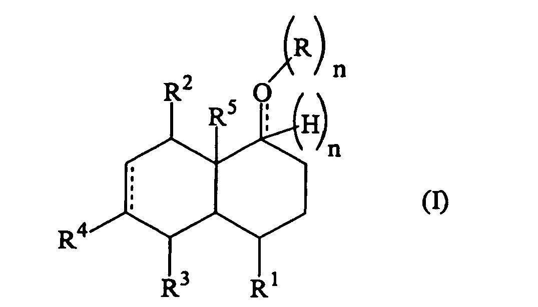 wherein R¹ is a lower alkyl, the R² to R⁵ are hydrogen atoms or lower alkyls and at least one dotted line is a double bond.

The present invention concerns also the compositions or articles associated with said compound.

### Prior art

The compounds of formula (I) are generally known from the prior art, where they are described, in general, in the context of chemical synthesis.

However, the prior art documents disclosing said compounds do not report or suggest any organoleptic properties of the compounds of formula (I), or any use of said compound in the field of perfumery.

In US 4377714, US 5114915 and 4671798 are disclosed decalinols or decalones having a quite different substitution patterns (at least a *gem*-diMe group) and which are described as being useful perfuming ingredients. In US 4311852 and US 4118343 are disclosed tricyclic derivatives of decalinols or decalones having a quite different structure and which are described as being useful perfuming ingredients. Again, said documents, separately or together, do not report or suggest any organoleptic properties of the compounds of formula (I), or any use of said compound in the field of perfumery.

### Description of the invention

We have now discovered that a compound of formula 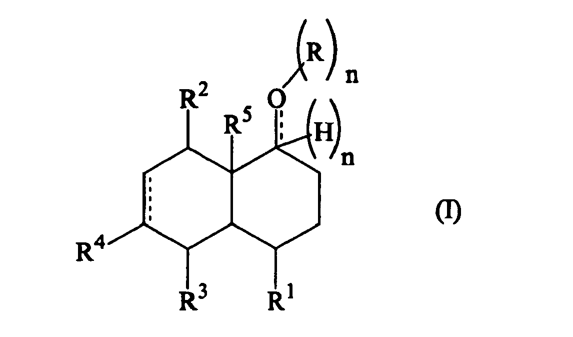 wherein the dotted lines represent a single or double bond and at least one of said dotted lines represents a double bond;
n represents simultaneously 0, in which case the oxygen atom is bounded to the cyclanic carbon atom by a double bond, or 1, in which case the oxygen atom is bounded to the cyclanic carbon atom by a single bond,
R represents a hydrogen atom or a HCO or MeCO group;
R¹ represents a linear or branched C₁-C₄ alkyl or alkenyl group;
R² represents a hydrogen atom or a methyl or ethyl group;
R³ represents, taken alone, a hydrogen atom or, taken together with R², a bridging CH₂ group;
R⁴ represents a hydrogen atom or a methyl or ethyl group; and
R⁵ represents a hydrogen atom or a linear or branched C₁-C₃ alkyl group;
is a useful perfuming ingredient, which is characterized by an odor of the woody type with optionally citrus notes.

The compound of formula (I) possesses several asymmetric carbon atoms. Therefore, the compound of formula (I) can be in the form of any one of its stereoisomers. Furthermore it is also understood that the invention's compound can be in the form of a mixture of any one of said stereoisomers.

Preferably, substituents R¹, R², R³, R⁴ and R⁵ have in total, i.e. all together, 3, 4 or 5 carbon atoms.

According to another embodiment of the invention, the compounds of formula (I) wherein the dotted lines, R³ and n are defined as above;
R represents a hydrogen atom or a MeCO group;
R¹ represents a methyl, ethyl or propyl group;
each R², R⁴ and R⁵ represent a hydrogen atom or a methyl group; and
the substituents R¹, R², R³, R⁴ and R⁵ have in total, i.e. all together, 3 or 4 carbon atoms; are particularly suitable of a large number of applications in fine or functional perfumery.

Furthermore, the compounds of formula 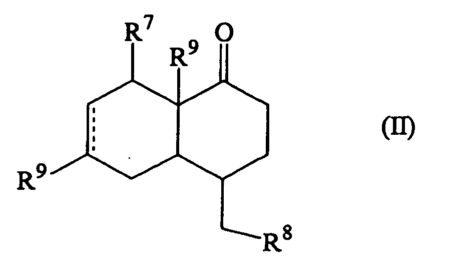 wherein the dotted lines represent a single or double bond;
R⁷ and R⁸ represent, each, a hydrogen atom or a methyl group;
R⁹ are both hydrogen atoms or are both methyl groups; and
   the substituents R⁷, R⁸ and R⁹ have in total, i.e. all together, 2 or 3 carbon atoms; or the compounds of formula 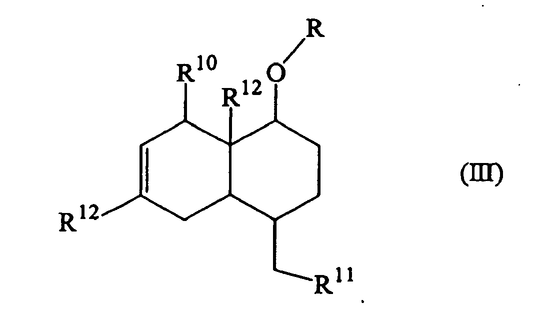 wherein R represents a hydrogen atom or a MeCO group;
R¹⁰ and R¹¹ represent, each, a hydrogen atom or a methyl group;
one R¹² is a hydrogen atom and the other is a methyl group; and
   the substituents R¹⁰, R¹¹ and R¹² have in total, i.e. all together, 2 or 3 carbon atoms;
   are very appreciated by the perfumers for their woody-vetiver/cedar or woody-grapefruit note. The compounds of formula (II) or (III), at the exception of 4,6,8,8a-tetramethyl-3,4,4a,5,8,8a-hexahydro-naphthalenone and 4,6,8a-trimethyl-3,4,4a,5,8,8a-hexahydro-1(2H)-naphthalenone and their isomers, are also new and are a further object of the present invention. The known compounds of formula (II) or (III) have been reported by C.Angell et all in J.Org.Chem. 1986, 51, 5177, wherein they were prepared for a NMR study of geminal di-methyl analogues.

Amongst the compounds responding to the formulae cited above, one may cite in particular, and as non-limiting example, 4,6,8a-trimethyl-3,4,4a,5,8,8a-hexahydro-1(2H)-naphthalenone. This compound has a scent characterized by a woody-cedar and ambery-leather note as well as a jute, patchouli and agarwood connotation. In fact 4,6,8a-trimethyl-3,4,4a,5,8,8a-hexahydro-1(2H)-naphthalenone has an odor which reminds of Vertofix^{®} (methyl cedryl ketone; origin: IFF, USA) but having a greater substantivity and volume than the odor of the latter.

The fragrance and performance of 4,8,8a-trimethyl-1,2,3,4,4a,5,8,8a-octahydro-1-naphthalenyl acetate are very similar to those of the above-cited 4,6,8a-trimethyl-3,4,4a,5,8,8a-hexahydro-1(2H)-naphthalenone, but distinguish themselves by the presence of an additional note of the ozone type.

Another example of invention's compound is perhydro-4-ethyl-8-methyl-1-naphthalenone that has an odor of the woody-vetiver type, with a strong connotation of the vetiveryl acetate, vetiverone type in which the woody, earthy and rooty notes are married in a very natural and elegant manner. It is very rare for a product to possess such a natural vetiver note.

Furthermore, as ketone one may also cite perhydro-8-methyl-4-propyl-1-naphthalenone which has a woody-vetiver note similar to that of perhydro-4-ethyl-8-methyl-1-naphthalenone but possesses also a well distinct grapefruit, rhubarbe note.

The scent of 4-ethyl-6,8-dimethyl-1,2,3,4,4a,5,8,8a-octahydro-1-naphthalenol has a quite distinct odor compared to the above-mentioned compounds. The odor of said naphthalenol is characterized by a woody-pin note and a clean, elegant, fresh and powerful citrus note, of the grapefruit type, which reminds of the odor of Nootkatone, a natural ingredient of pink-grapefruit peel scent. Furthermore, 4-ethyl-6,8-dimethyl-1,2,3,4,4a,5,8,8a-octahydro-1-naphthalenol possesses a fragrance having an astonishing substantivity, which is rare for this type of note.

Contrary to the above-mentioned alcohol, the tri-cyclic alcohol 2,6-dimethyltricyclo[6.2.1.0(2,7)]undec-9-en-3-ol possesses a woody odor which is typically of the patchouli and rooty type.

Finally, one may also cite the perhydro-4-ethyl-6,8a-dimethyl-1-naphthalenone and its olefinic analogue 4-ethyl-6,8a-dimethyl-3,4,4a,5,8,8a-hexahydro-1(2H)-naphthalenone. Both compounds have a scent similar to that of perhydro-4-ethyl-8-methyl-1-naphthalenone, but are a bit less strong and have also a grapefruit note, which is stronger for the perhydro-4-ethyl-6,8a-dimethyl-1-naphthalenone.

As mentioned above, the invention concerns also the use of a compound of formula (I) as perfuming ingredient. In other words it concerns a method to confer, enhance, improve or modify the odor properties of a perfuming composition or of a perfumed article, which method comprises adding to said composition or article an effective amount of at least a compound of formula (I). By "use of a compound of formula (I)" it has to be understood here also the use of any composition containing the compound (I) and which can be advantageously employed in the perfumery industry as active ingredients.

Said compositions, which are in fact perfuming compositions that can be advantageously employed as perfuming ingredients, are also an object of the present invention.

Therefore, another object of the present invention is a perfuming composition comprising:
i) as perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" we mean here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used.

As solid carrier one may cite, as non-limiting examples, absorbing gums or polymers, or yet encapsulating materials. Examples of such materials, for example, may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloids : Stabilisatoren, Dickungs- und Gehermittel in Lebensmittel, Band 2 der Schriftenreihe Lebensmittelchemie, Lebensmittelqualität, Behr's VerlagGmbH & Co., Hamburg, 1996. The encapsulation is a well known process to a person skilled in the art, and may be performed, for instance, using techniques such as spray-drying, agglomeration or yet extrusion; or consists of a coating encapsulation, including coacervation and complex coacervation techniques.

Generally speaking, by "perfumery base" we mean here a composition comprising at least one perfuming co-ingredient.

Said perfuming co-ingredient is not of the formula (I). Moreover, by "perfuming co-ingredient" it is meant here a compound, which is used in perfuming preparation or composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of its general knowledge and according to intended use or application and the organoleptic effect desired. In general terms, these perfuming co-ingredients belong to chemical classes as varied as alcohols, aldehydes, ketones, esters, ethers, acetates, nitriles, terpene hydrocarbons, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

For the compositions which comprise both a perfumery carrier and a perfumery base, other suitable perfumery carrier, than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company).

Generally speaking, by "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

An invention's composition consisting of at least one compound of formula (I) and at least one perfumery carrier represents a particular embodiment of the invention as well as a perfuming composition comprising at least one compound of formula (I), at least one perfumery carrier, at least one perfumery base, and optionally at least one perfumery adjuvant.

It is useful to mention here that the possibility to have, in the compositions mentioned above, more than one compound of formula (I) is important as it enables the perfumer to prepare accords, perfumes, possessing the odor tonality of various compounds of the invention, creating thus new tools for their work.

Its is also understood here that any mixture resulting directly from a chemical synthesis, e.g. without an adequate purification, in which the compound of the invention would be involved as a starting, intermediate or end-product could not be considered as a perfuming composition according to the invention.

Furthermore, the invention's compound can also be advantageously used in all the fields of modem perfumery to positively impart or modify the odor of a consumer product into which said compound (I) is added. Consequently, a perfumed article comprising:
i) as perfuming ingredient, at least one compound of formula (I) as defined above; and
ii) a consumer product base,
is also an object of the present invention.

For the sake of clarity, it has to be mentioned that, by "consumer product base" we mean here a consumer product which is compatible with perfuming ingredients. In other words, a perfumed article according to the invention comprises the functional formulation, as well as optionally additional benefit agents, corresponding to a consumer product, e.g. a detergent or an air freshener, and an olfactive effective amount of at least one invention's compound.

The nature and type of the constituents of the consumer product do not warrant a more detailed description here, which in any case would not be exhaustive, the skilled person being able to select them on the basis of its general knowledge and according to the nature and the desired effect of said product.

Examples of suitable consumer products include solid or liquid detergents and fabric softeners as well as all the other articles common in perfumery, namely perfumes, colognes or after-shave lotions, perfumed soaps, shower or bath salts, mousses, oils or gels, hygiene products or hair care products such as shampoos, body-care products, deodorants or antiperspirants, air fresheners and also cosmetic preparations. As detergents there are intended applications such as detergent compositions or cleaning products for washing up or for cleaning various surfaces, e.g. intended for textile, dish or hard-surface treatment, whether they are intended for domestic or industrial use. Other perfumed articles are fabric refreshers, ironing waters, papers, wipes or bleaches.

Some of the above-mentioned consumer product bases may represent an aggressive medium for the invention's compound, so that it may be necessary to protect the latter from premature decomposition, for example by encapsulation.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0.01 % to 25 % by weight, or even more, of the compounds of the invention based on the weight of the composition into which they are incorporated. Concentrations lower than these, such as in the order of 1% to 10% by weight, can be used when these compounds are incorporated into perfumed articles.

The use and article comprising an invention's compound selected from the group consisting of the 4,6,8a-trimethyl-3,4,4a,5,8,8a-hexahydro-1(2H)-naphthalenone, 4-ethyl-6,8-dimethyl-1,2,3,4,4a,5,8,8a-octahydro-1-naphthalenol, perhydro-4-ethyl-8-methyl-1-naphthalenone and perhydro-8-methyl-4-propyl-1-naphthalenone are a preferred embodiment of the invention. Three of said compounds are also new and are also an object of the present invention.

The invention's compounds can be prepared by a process involving a Lewis acidic catalyzed Diels-Alder reaction between a diene of formula (IV) and a dienophyle of formula (V), 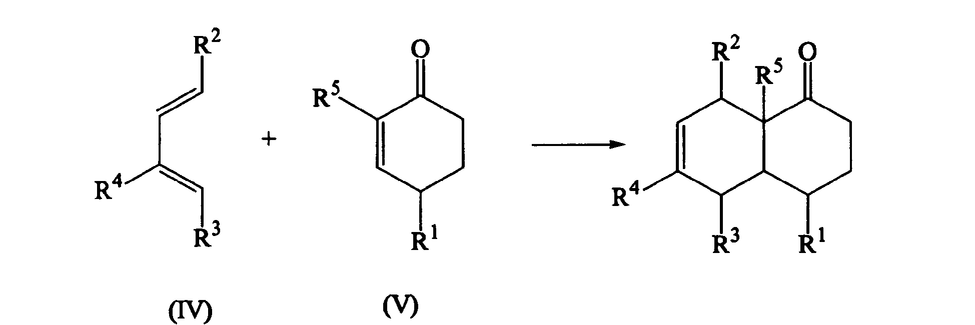 wherein the symbols have the meaning indicated above, and then optionally a reduction of the carbonyl or of the carbon-carbon double bond. Specific examples are given further below.

The invention will now be described in further detail by way of the following examples, wherein the abbreviations have the usual meaning in the art, the temperatures are indicated in degrees centigrade (°C) ; the NMR spectral data were recorded in CDCl₃ (if not stated otherwise) with a 360 or 400 MHz machine for ¹H and ¹³C, the chemical displacements δ are indicated in ppm with respect to TMS as standard, the coupling constants J are expressed in Hz.

### Example 1

### Synthesis of compounds of formula (I)

The 4-ethyl-cyclohex-2-enone is a known compound (see Duhamel et al in Tetrahedron, 1986, 42, 4777) as well as 2,4-dimethyl-cyclohex-2-enone (see Blanc et al in Helv.Chim.Acta, 1964, 725).

### 1) Synthesis of the starting cyclohexenones

### General procedure for the first step

In an appropriate vessel were introduced the aldehyde, the vinyl-ketone, diethylamine, and toluene and the mixture was heated 20 hours at 90°C under good stirring. Afterwards, the temperature was dropped to 25°C and the reaction mixture was hydrolyzed with 5% aqueous HCl and extracted twice with Et₂O. The organic layer was then washed with a saturated NaHCO₃ aqueous solution, water, brine and then dried over Na₂SO₄. Evaporation of the solvents and distillation provided the end product.

### a) 2-Ethyl-5-oxoheptanal

| | |
|---|---|
| Starting materials and quantities: | Butyraldehyde (400 mmol) |
| | Ethylvinylketone (600 mmol) |
| | Diethylamine (80 mmol) |
| | Toluene (200 ml) |

Was obtained with a yield of 80%.
B.p. = 85°C at 1,1 mbar
MS: 156 (M+, 2); 138 (6); 128 (12); 127 (14); 109 (10); 99 (30); 85 (28); 81 (41); 72 (60); 57 (100); 55 (40).
¹H-NMR: 0.95 (t, J = 7, 3 H); 1.05 (t, J = 7, 3 H); 1.40-1.92 (m, 4 H); 2.12-2.25 (m, 1 H); 2.32-2.52 (m, 4 H), 9.57 (d, J = 2.4 Hz, I H).

### b) 5-Oxo-2-propylheptanal

| | |
|---|---|
| Starting materials and quantities: | Valeraldehyde (630 mmol) |
| | Ethylvinylketone (950 mmol) |
| | Diethylamine (130 mmol) |
| | Toluene (400 ml) |

Was obtained with a yield of 97%.
B.p. = 93°C at 1,7 mbar
MS: 170 (M+, 3); 152 (5); 141 (21); 123 (18); 113 (33); 99 (11); 95 (45); 85 (22); 81 (13); 72 (72); 57 (100); 55 (37)
¹H-NMR: 0.92 (t, J = 7, 3 H); 1.04 (d, J = 7, 3 H); 1.30-1.47 (m, 3 H); 1.60-1.93 (m, 3 H); 2.20-2.32 (m, 1 H); 2.35-2.52 (m, 4 H); 9.54 (d, J = 2.4 Hz, I H).

### c) 5-Oxo-2-propylhexanal

| | |
|---|---|
| Starting materials and quantities: | Valeraldehyde (0.8 mol) |
| | Methylvinylketone (1.2 mol) |
| | Diethylamine (0.16 mol) |
| | Toluene (500 ml) |

Was obtained with a yield of 93 %.
B.p. = 94 °C at 1,1 mbar.
MS: 156 (M+; 1); 138 (5); 127 (15); 114 (9); 109 (10); 99 (9); 95 (13); 86 (20); 81 (12); 71 (18); 58 (100); 43 (93).
¹H-NMR: 0.92 (t, J = 7 Hz, 3 H); 1.30-1.48 (m, 3 H) ; 1.60-1.95 (m, 3 H) ; 2.13 (s, 3 H) ; 2.22-2.32 (m, 1 H); 2.38-2.55 (m, 2 H); 9.55 (d, J = 2.4 Hz, I H).

### General procedure for the second step

In an appropriate vessel were introduced aqueous KOH and tetramethylammonium hydroxide in THF. Then the compound obtained in the first step, in Et₂O, was added dropwise and the mixture stirred 2 hours, at room temperature. When the reaction ended, the mixture was hydrolyzed with 5% aqueous HCl and extracted twice with Et₂O. The organic layer was then washed with a saturated NaHCO₃ aqueous solution, water, brine and then dried over Na₂SO₄. Evaporation of the solvents and distillation provided the end product.

### a) 4-Ethyl-2-methyl-2-cyclohexen-1-one

| Starting materials and quantities: | |
|---|---|
| | the compound obtained in the first step (a) (440 mmol) |
| | Teramethylammonium hydroxide (37 mmol) |
| | KOH (90 mmol), in 100 ml of water |
| | THF (100 ml) |
| | Et₂O (100 ml) |

Was obtained with a yield of 93%.
B.p. = 89°C at 3,0 mbar
MS: 138 (M+, 94); 123 (8); 109 (20); 96 (86); 95 (60); 81 (100); 79 (30); 69 (19); 67 (29)
¹H-NMR: 1.01 (t, J = 7, 3 H); 1.38-1.60 (m, 3 H); 1.75 (broad s, 3 H); 2.02-2.12 (m, 1 H); 2.25-2.38 (m, 2 H); 2.50 (m, 1 H); 6.62 (m, I H).

### b) 2-Methyl-4-propyl-2-cyclohexen-1-one

| Starting materials and quantities: | |
|---|---|
| | the compound obtained in the first step (b) (610 mmol) |
| | Teramethylammonium hydroxide (55 mmol) |
| | KOH (180 mmol), in 200 ml of water |
| | THF (200 ml) |
| | Et₂O (200 ml) |

Was obtained with a yield of 86%.
B.p. = 93°C at 2,5 mbar
MS: 152 (M+, 76); 123 (10); 110 (41); 109 (23); 95 (100); 82 (85); 81 (60); 79 (25); 69 (19); 67 (25).
¹H-NMR: 0.93 (t, J = 7, 3 H); 1.30-1.50 (m, 4 H); 1.55-1.70 (m, 1 H); 1.75 (broad s, 3H); 2.02-2.14 (m, 1 H); 2.27-2.45 (m, 2 H); 2.50 (m, 1 H); 6.60 (m, 1 H).

### c) 3-Propyl-2-cyclohexen-1-one

| Starting materials and quantities: | |
|---|---|
| | the compound obtained in the first step (c) (0.764 mol) |
| | Tetramethylammonium hydroxide pentahydrate (0.066 mol) |
| | Potassium hydroxide (10 g, 0.15 mol) in water (200 ml) |
| | THF (200 ml) |
| | Diethylether (200 ml) |

Was obtained with a yield of 82 %.
B.p. = 84 °C at 2,5 mbar.
MS: 138 (M+, 52); 110 (28); 96 (43); 81 (100); 53 (17); 41 (17).
¹H-NMR 0.95 (t, J = 7, 3 H) ; 1.20-1.73 (5 H) ; 2.05-2.20 (m, I H) ; 2.28-2.53 (m, 3 H) ; 5.96 (dd, J₁ = 10.3 Hz, J₂= 2.4 Hz, 1 H) ; 6.87 (m, 1 H).

### 2) Synthesis of the compounds of formula (I)

### I) General procedure for the Diels-Alder coupling

In a 500 ml reactor were introduced the AlEtCl₂, or the AlCl₃, 0.1 g of BHT and toluene, or CH₂Cl₂. Then, under vigorous stirring, was added the appropriate cyclohexenone dropwise, so as to maintain the temperature below 30°C. Afterward, was added the diene dropwise and when the reaction ended the reaction mixture was hydrolyzed with 5% aqueous HCl, extracted twice with Et₂O. The organic layer was then washed with a saturated NaHCO₃ aqueous solution, water, brine and then dried over Na₂SO₄. Evaporation of the solvents, chromatography (SiO₂, elution heptane/AcOEt 98:2) and distillation provided the end product.

### a) 4,6,8a-Trimethyl-3,4,4a,5,8,8a-hexahydro-1(2H)-naphthalenone

| | |
|---|---|
| Starting materials and quantities: | 2,4-Dimethyl-2-cyclohexen-1-one (320 mmol) |
| | AlCl₃ (80 mmol) |
| | Isoprene (4,8 mol) |
| | Toluene (500 ml) |

Was obtained with a yield of 83%, in the form of a mixture of isomers (88/4/9).
B.p. = 78°C at 0,023 mbar
MS (major isomer): 192 (M+, 14); 177(32); 159 (44); 149 (100); 132 (36); 119 (21); 93 (33); 91 (33); 77 (16).
¹H-NMR: 0.94 (d, J = 7, 3 H); 1.10 (s, 3 H); 1.25-1.43 (m, 3 H); 1.58-2.80 (m, 7 H); 1.67 (broad s, 3 H); 5.25-5.35 (m, 1 H).

### b) 4-Ethyl-6,8a-dimethyl-3,4,4a,5,8,8a-hexahydro-1(2H)-naphthalenone

| | |
|---|---|
| Starting materials and quantities: | 4-Ethyl-2-methyl-2-cyclohexen-1-one (109 mmol) |
| | AlCl₃ (27 mmol) |
| | Isoprene (218 mmol) |
| | Toluene (150 ml) |

Was obtained with a yield of 68%, in the form of a mixture of isomers (93/2/5).
B.p. = 85°C at 0,004 mbar
MS (major isomer): 206 (M+, 11); 191 (25); 173 (12); 163 (100); 159(39); 132 (19); 119 (14); 107 (23); 93 (24); 91 (24).
¹H-NMR: 0.87 (t, J = 7, 3 H); 1.08 (s, 3 H); 1.13-1.70 (m, 6 H); 1.18 (broad s, 3 H); 1.97-2.07 (m, 3 H); 2.30 (m, 1 H); 2.40 (m, 1 H); 2.70 (m, 1 H); 5.32 (m, 1 H).

### c) 4-Ethyl-8-methyl-3,4,4a,5,8,8a-hexahydro-1(2H)-naphthalenone

| | |
|---|---|
| Starting materials and quantities: | 4-Ethyl-2-cyclohexen-1-one (144 mmol) |
| | EtAlCl₂ (60 mmol) |
| | Piperylene (288 mmol) |
| | CH₂Cl₂ (150 ml) |

Was obtained with a yield of 60%, in the form of a mixture of isomers (20/7/81/90).
B.p. = 85°C at 0,042 mbar
MS (major isomer): 192 (M+, 59); 177 (17); 163 (21); 145 (100); 133 (34); 121 (49); 93 (79); 79 (48); 77 (42).
¹H-NMR: 0.85-1.03 (m, 3 H); 1.15-1.55 (m, 5 H); 1.60-2.50 (m, 9 H); 2.72-2.92 (m, 1 H); 5.42-5.60 (m, 2 H).

### d) 4-Ethyl-6,8-dimethyl-3,4,4a,5,8,8a-hexahydro-1(2H)-naphthalenone

| | |
|---|---|
| Starting materials and quantities: | 4-Ethyl-2-cyclohexen-1-one (80 mmol) |
| | EtAlCl₂ (40 mmol) |
| | Methylpentadiene (160 mmol) |
| | CH₂Cl₂ (200 ml) |

Was obtained with a yield of 68%, in the form of a mixture of isomers (19/78/3).
B.p. = 105°C at 0,031 mbar
MS (major isomer): 206 (M+, 88); 191 (63); 177 (15); 173 (17); 159 (91); 147 (51); 135 (73); 119 (34); 107 (100); 91 (60).
¹H-NMR: 0.85-1.05 (m, 6 H); 1.20 (m, 3 H); 1.40-2.85 (m, 12 H); 5.14-5.43 (m, 1 H).
Odor: woody, rhubarb, balsamic and grapefruit with some bottom notes of the earthy, humid or marine type

### e) (1RS,2RS,6RS,7SR,8SR)-2,6-dimethyltricyclo[6.2.1.0(2,7)]undec-9-en-3-one

| | |
|---|---|
| Starting materials and quantities: | 2,4-Dimethyl-2-cyclohexen-1-one (91 mmol) |
| | EtAlCl₂ (45,5 mmol) |
| | Cyclopentadiene (182 mmol) |
| | CH₂Cl₂ (100 ml) |

Was obtained with a yield of 61%, in the form of a mixture of isomers (14/79/2/4).
B.p. = 88°C at 0,024 mbar
MS (major isomer): 125 (100); 107 (8); 91 (11); 66 (96).
¹H-NMR: 0.98-1.10 (m, 6 H); 1.12-2.00 (m, 5 H); 2.20-3.20 (m, 5 H); 5.92-6.40 (m, 2 H).
Odor: woody, camphoraceous and champhene

### f) 4,8,8a-Trirnethyl-3,4,4a,5,8,8a-hexahydro-1(2H)-naphthalenone

| | |
|---|---|
| Starting materials and quantities: | 2,4-Dimethyl-2-cyclohexen-1-one (60 mmol) |
| | EtAlCl₂ (60 mmol) |
| | Piperylene (450 mmol) |
| | CH₂Cl₂ (100 ml) |

Was obtained with a yield of 70%, in the form of a mixture of isomers (58/42).
B.p. = 82°C at 0,023 mbar
MS (major isomer): 192 (M+, 20); 177 (10); 149 (21); 135 (11); 125 (100); 107 (33); 93 (19); 91 (22); 68 (49).
MS (minor isomer): 192 (86); 177 (32); 149 (33); 135 (37); 125 (92); 107 (100); 93 (46); 91 (51); 68 (60).
¹H-NMR: 0.75-1.05 (m, 6 H); 1.18-1.48 (m, 5 H); 1.65-2.80 (m, 7 H); 5.35-5.62 (m, 2 H).
Odor: woody-camphor and eucalyptus

### g) 6,8a-Dimethyl-4-propyl-3,4,4a,5,8,8a-hexahydro-1(2H)-naphthalenone

| | |
|---|---|
| Starting materials and quantities: | 4-Propyl-2-methyl-2-cyclohexen-1-one (109 mmol) |
| | AlCl₃ (27 mmol) |
| | Isoprene (218 mmol) |
| | Toluene (150 ml) |

Was obtained with a yield of 37%, in the form of a mixture of isomers (92/4/4).
B.p. = 81°C at 0,045 mbar
MS (major isomer): 220 (M+, 15); 205 (29); 202 (14); 177 (100); 159 (48); 135 (37); 132 (36); 119 (18); 107 (27); 93 (30); 91 (31).
¹H-NMR: 0.92 (t, J = 7, 3 H); 1.08 (s, 3 H); 1.10-1.70 (m, 9 H); 1.68 (broad s, 3 H); 2.05 (m, 2 H); 2.30 (m, 1 H); 2.40 (m, 1 H); 2.70 (m, 1 H); 5.32 (m, 1 H).
Odor: woody-vetiver and grapefruit-nootkatone

### h) 4,6,8,8a-Tetramethyl -3,4,4a,5,8,8a-hexahydro-1(2H)-naphthalenone

| | |
|---|---|
| Starting materials and quantities: | 2,4-Dimethyl-2-cyclohexen-1-one (58 mmol) |
| | EtAlCl₂ (29 mmol) |
| | Methylpentadiene (117 mmol) |
| | CH₂Cl₂ (150 ml) |

Was obtained with a yield of 90%, in the form of a mixture of isomers (89/11).
B.p. = 85°C at 0,065 mbar
MS (major isomer): 206 (M+, 16); 191 (8); 177 (10); 73 (8); 163 (23); 125 (43); 121 (22); 107 (19); 105 (13); 91 (16); 82 (100); 67 (36).
¹H-NMR: 0.70-1.20 (m 9 H); 1.30-2.80 (m, 12 H); 5.05-5.40 (m, 1 H). Odor: woody, naphthalene and grapefruit

### i) 8-Methyl-4-propyl-3,4,4a,8,8a-hexahydro-1(2H)-naphthalenone

| | |
|---|---|
| Starting materials and quantities: | 4-Propyl-2-cyclohexen-1-one (0.1 mol) |
| | Ethyl aluminium dichloride (20 ml, 0.02 mol) |
| | Piperylene (0.2 mol) |
| | CH₂Cl₂ (150 ml). |

Was obtained with a yield of 52 %, in the form of a mixture of isomers (10/3/45/42).
B.p. = 80 °C at 0,021 mbar
MS (major isomer): 206 (M+, 49); 191 (14); 177 (7); 163 (15); 145 (100); 136 (20); 121 (59); 105 (47); 93 (81); 79 (51); 77 (42); 67 (23); 55 (34); 41 (36).
¹H-NMR: 0.88-1.02 (m, 3 H); 1.20 (m, 3 H); 1.25-2.50 (m, 13 H); 2.72-2.92 (m, 1 H); 5.45-5.60 (m, 2 H).

### II) General procedure for the reduction of the ketone into the alcohol

In a 100 ml flask, maintained under Ar atmosphere, were introduced 2 molar equivalents, with respect of the ketone, of LiAlH₄ in l'Et₂O. Then the appropriate naphthalenone was added dropwise, so as to maintain the reflux. After completion of the reaction the mixture was stirred for 30 minutes at reflux. Afterwards the reaction mixture was hydrolyzed with a stoechiometric amount of aqueous NaOH and the organic layer was dried over Na₂SO₄. Evaporation of the solvents and distillation provided the end product.

### a) 4,8,8a-Trimethyl-1,2,3,4,4a,5,8,8a-octahydro-1- naphthalenol

| | |
|---|---|
| Starting materials and quantities: | Naphthalenone obtained under I.f) (78 mmol) |
| | Et₂O (150 ml) |

Was obtained with a yield of 79%, in the form of a mixture of isomers (59/34/7).
B.p. = 72°C at 0,002 mbar
MS (major isomer): 194 (M+, 2); 176 (45); 161 (34); 147 (11); 135 (18); 133 (13); 125 (39); 119 (100); 109 (91); 93 (49); 91 (41).
MS (minor isomer): 194 (M+, 0.2); 176 (26); 161 (21); 119 (100); 107 (19); 105 (38); 93 (24); 91 (23).
¹H-NMR: 0.78-1.30 (m, 10 H); 1.55-2.65 (m, 9 H); 3.30-3.90 (m, 1 H); 5.22-5.90 (m, 2 H).
Odor: woody, patchouli and earthy

### b) 2,6-Dimethyltricyclo[6.2.1.0(2,7)]undec-9-en-3-ol

| | |
|---|---|
| Starting materials and quantities: | Naphthalenone obtained under I.e) (21 mmol) |
| | Et₂O (400 ml) |

Was obtained with a yield of 89%, in the form of a mixture of isomers (18/66/10/4/2).
B.p. = 81°C at 0,008 mbar
MS (major isomer): 192 (M+, 0.1); 125 (95); 111 (47); 109 (70); 93 (27); 91 (23); 84 (38); 82 (28); 66 (100).
¹H-NMR: 0.55-0.95 (m, 6 H); 0.95-1.30 (m, 3 H); 1.35-2.25 (m, 6 H); 2.45-2.90 (m, 2H); 3.65-3.95 (m, 1 H); 6.02-6.45 (m, 2 H).

### c) 4,6,8,8a-Tetramethyl-1,2,3,4,4a,5,8,8a-octahydro-1-naphthalenol

| | |
|---|---|
| Starting materials and quantities: | Naphthalenone obtained under I.h) (36 mmol) |
| | Et₂O (50 ml) |

Was obtained with a yield of 98%, in the form of a mixture of isomers (25/75).
B.p. = 89°C at 0,039 mbar
MS (major isomer): 208 (M+, 4); 190 (39); 175 (36); 133 (88); 119 (41); 107 (39); 91 (30); 82 (100); 67 (38).
¹H-NMR: 0.75-1.25 (m, 10 H); 1.35-2.15 (m, 12 H); 3.30-3.55 (m, 1 H); 4.95-5.35 (m, 1 H).
Odor: woody-cedar with bottom notes of the dry pine forest type

### d) 4-Ethyl-6,8-dimethyl-1,2,3,4,4a,5,8,8a-octahydro-1-naphthalenol

| | |
|---|---|
| Starting materials and quantities: | Naphthalenone obtained under I.d) (73 mmol) |
| | Et₂O (150 ml) |

Was obtained with a yield of 89%, in the form of a mixture of isomers (3/10/62/5/11/5/3).
B.p. = 89°C at 0,030 mbar
MS (major isomer): 208 (M+, 5); 190 (41); 175 (23); 161 (40); 147 (10); 133 (19); 119 (100); 105 (81); 91 (28).
¹H-NMR: 0.80-1.10 (m, 6 H); 1.15-1.70 (m, 11 H); 1.75-2.50 (m, 5 H); 3.70-4.10 (m, 1 H); 5-05-5.50 (m, 1 H)

### III) General procedure for the hydrogenation of the naphthalenone into the perhydro naphthalenone

In a 100 ml flask were introduced the appropriate naphthalenone, ethyl acetate and 10% w/w, relative to the naphthalenone, of Pd/C 5%. The mixture was thus stirred under H₂, at a room temperature, until consumption of the theoretical amount of hydrogen. Afterwards, the reaction mixture was filtered over Nylon 6/6. Evaporation of the solvents and distillation provided the end product.

### a) Perhydro-4-ethyl-8-methyl-1-naphthalenone

| | |
|---|---|
| Starting materials and quantities: | Naphthalenone obtained under I.c) (52 mmol) |
| | Ethyl acetate (100ml) |
| | H₂(1,16 l) |

Was obtained with a yield of 95%, in the form of a mixture of isomers (35/51/1/12/1). B.p. = 87°C at 0,050 mbar
MS (major isomer): 194 (M+, 28); 165 (30); 151 (14); 138 (14); 125 (100); 110 (11); 95 (30).
MS (minor isomer): 194 (M+, 69); 179 (21); 165 (26); 151 (48); 138 (51); 125 (100); 123 (52); 110 (35); 95 (66).
¹H-NMR: 0.78-1.00 (m, 5 H); 110 (m, 3 H); 110-2.65 (m, 14 H).

### b) Perhydro-4-ethyl-6,8a-dimethyl-1-naphthalenone

| | |
|---|---|
| Starting materials and quantities: | Naphthalenone obtained under I.b) (29 mmol) |
| | Ethyl acetate (60 ml) |
| | H₂ (650 ml) |

Was obtained with a yield of 95%, in the form of a mixture of isomers (6/1/37/2/48/6).
B.p. = 95°C at 0,050 mbar
MS (major isomer): 208 (M+, 9); 193 (44); 139 (100); 109 (26); 95 (19); 81 (17).
MS (minor isomer): 208 (M+, 2); 139 (100); 109 (17); 95 (12); 81 (12).
¹H-NMR: 0.80-1.00 (m, 7 H); 1.15-1.25 (m, 5 H); 1.25-1.75 (m, 8 H); 1.85-2.08 (m 2 H); 2.15-2.35 (m, 1 H); 2.48-2.70 (m, 1 H).

### c) Perhydro-8-methyl-4-propyl-1-naphthalenone

| | |
|---|---|
| Starting materials and quantities: | Naphthalenone obtained under I.i) (0.058 mol) |
| | Ethyl acetate (120 ml) |
| | H₂(1.3 l) |

Was obtained with a yield of 96%, in the form of a mixture of isomers (17/70/5/6/2).
B.p. = 85 °C at 0,016 mbar.
MS (major isomer): 208 (M+, 50); 190 (5); 179 (40); 165 (21); 139 (100); 123 (30); 110 (97); 95 (55); 81 (29); 67 (23); 55 (33); 41 (23).
¹H-NMR: 0.85-1.00 (m, 6 H); 1.10-2.70 (m, 18H).

### IV) General procedure for the esterification of the alcohol

In a 250 ml flask were introduced the appropriate alcohol, CH₂Cl₂, dimethylamminopyridine, pyridine and the appropriate carboxylic anhydride. The mixture was thus stirred 24 hours at room temperature. When the reaction has finished the reaction mixture was hydrolyzed with 5% aqueous HCl, extracted twice with Et₂O. The organic layer was then washed an aqueous solution of CuSO₄, a saturated NaHCO₃ aqueous solution, water, brine and then dried over Na₂SO₄. Evaporation of the solvents provided the end product.

### a) 4,8,8a-Trimethyl-1,2,3,4,4a,5,8,8a-octahydro-1-naphthalenyl acetate

| | |
|---|---|
| Starting materials and quantities: | Alcohol obtained under II.a) (46 mmol) |
| | CH₂Cl₂ (100 ml) |
| | Acetic anhydride (69 mmol) |
| | Dimethylamminopyridine (4.6 mmol) |
| | Pyridine (78 mmol) |

Was obtained with a yield of 83%, in the form of a mixture of isomers (26/60/33/11). B.p. = 69°C at 0,008 mbar
MS (major isomer): 236 (M+, 0.1); 194 (2); 176 (57); 161 (37); 147 (15); 133 (17); 119 (75); 108 (100); 93 (69).
¹H-NMR: 0.78-1.30 (m, 11 H); 1.35-2.35 (m, 10 H); 4.57-4.97 (m, 1 H); 5.35-5.90 (m, 2 H).

### b) 4,6,8,8a-Tetramethyl-1,2,3,4,4a,5,8,8a-octahydro-1-naphthalenyl formate

| | |
|---|---|
| Starting materials and quantities: | Alcohol obtained under II.c) (3.8 mmol) |
| | CH₂Cl₂ (20 ml) |
| | Acetic anhydride (11.5 mmol) and formic acid |
| | (13,8 mmol) heated 2 hours at 55°C |
| | Dimethylamminopyridine (not used) |
| | Pyridine (not used). |

Was obtained with a yield of 98%, in the form of a mixture of isomers (7/19/74).
B.p. = 74°C at 0,008 mbar
MS (major isomer): 236 (M+, 0.2); 190 (30); 175 (25); 133 (52); 121 (19); 119 (26); 107 (28); 105 (15); 82 (100)
¹H-NMR: 0.80 (m, 3 H); 0.85-1.30 (m, 8 H); 1.40-2.20 (m, 10 H); 4.70-4.88 (m, 1 H); 4.96-5.30 (m, 1 H); 7-97-8.17 (m, 1 H).
Odor: woody, ambery and vetiveryl acetate

### Example 2

### Preparation of a perfuming composition

An eau de toilette for man, of the citrus type, was prepared by admixing the following ingredients:

| Ingredient | Parts by weight |
|---|---|
| Geranyl acetate | 10 |
| Linalyl acetate | 420 |
| 10%* Raspberry ketone | 20 |
| Bergamote essential oil | 200 |
| Citral | 30 |
| Lemon essential oil | 500 |
| 50%** Galaxolide^{®1)} | 100 |
| 10%* Galbanum essential oil | 90 |
| Clove essential oil | 90 |
| Lavander essential oil | 200 |
| Linalool | 70 |
| Lyral^{®2)} | 340 |
| Sweet marjoram essential oil | 120 |
| Mousse Chêne | 50 |
| Nutmeg essential oil | 160 |
| 3-(Iso-camphyl-5)-cyclohexanol | 200 |
| Ylang Extra | 100 |
| | 2700 |

| | |
|---|---|
| * in dipropyleneglycol ** in isopropyl myristate 1) 1,3,4,6,7,8-hexahydro-4,6,6,7,8,8-hexamethyl-cyclopenta-g-2-benzopyrane; origin: International Flavors & Fragrances, USA 2) 4/3-(4-hydroxy-4-methylpentyl)-3-cyclohexene-1-carbaldehyde; origin: International Flavors & Fragrances, USA | |

The addition of 800 parts by weight of 4,6,8a-trimethyl-3,4,4a,5,8,8a-hexahydro-1(2H)-naphthalenone to the above-described eau de toilette imparted to the fragrance of the latter a remarkably natural and strong connotation of the woody, cedar, Vertofix^{®}, cedryl acetate, which was very warm and had a lot of volume.

When instead of the above mentioned compound was added the same amount of 4,8,8a-trimethyl-1,2,3,4,4a,5,8,8a-octahydro-1-naphthalenyl acetate the effect obtained was quite similar although slightly less round.

The effect obtained by the addition of 800 parts by weight of perhydro-4-ethyl-8-methyl-1-naphthalenone to the above-described eau de toilette was quite different from the one described for the two compounds mentioned above. The new fragrance thus obtained had now a scent much more in the direction of vetiver, a bit watery and very pleasant and noble.

Also the effect obtained by the addition of 800 parts by weight of 4-ethyl-6,8-dimethyl-1,2,3,4,4a,5,8,8a-octahydro-1-naphthalenol to the above-described fine perfumery composition was very different from the one described for the other compounds mentioned above. The scent became almost of the grapefruit type with a woody bottom note. The grapefruit note was devoid of the classical grapefruit-sulfury note and was reminiscent of nootkatone.

### Example 3

### Preparation of a perfuming composition

A perfuming composition of the citrus type, for a powder detergent, was prepared by admixing the following ingredients:

| Ingredient | Parts by weight |
|---|---|
| 1,1-Dimethyl-2-phenylethyl acetate | 20 |
| Citronellyl acetate | 20 |
| Terpenyl acetate | 300 |
| Isobornyl acetate | 20 |
| 10%* Fenchylic alcool | 20 |
| Aldehyde C 12 | 20 |
| 10%* 4-Ethyl-benzaldehyde | 10 |
| Aldehyde MNA | 10 |
| Methyl Anthranilate | 60 |
| Benzylacetone | 20 |
| Borneol | 10 |
| Cashmeran ^{®1)} | 100 |
| Cetalox ^{® 2)} | 100 |
| Citronellol | 810 |
| 10%* Coumarine | 40 |
| (-)-(1'R,E)-2-Ethyl-4-(2',2',3'-trimethyl-3'-cyclopenten-1'-yl)-2-buten-1-ol-³⁾ | 20 |
| Dihydromyrcenol | 2000 |
| Geranyl Nitrile | 30 |
| Hédione ^{®4)} | 100 |
| Heliotropine | 30 |
| Ionone alpha | 10 |
| Iralia^{®5)}Total | 20 |
| Lavandin Grosso | 50 |
| Mayol^{®6)} | 30 |
| 10%* Crystalmoss | 20 |
| Neobutenone ^{® 7)} | 20 |
| Nonalactone gamma | 10 |
| Rose oxide | 10 |
| Phenylhexanol | 250 |
| Verdyl propionate | 380 |
| Isobornyl propionate | 300 |
| 10%* Romascone ^{®8)} | 20 |
| Terpineol | 40 |
| Linalool | 70 |
| Undecavertol ^{® 9)} | 10 |
| Verdox ^{®10)} | 1500 |
| Yara Yara | 20 |
| | 6500 |

| | |
|---|---|
| * in dipropyleneglycol 1) 1,2,3,5,6,7-hexahydro-1,1,2,3,3-pentamethyl-4-indenone; origin: International Flavors & Fragrances, USA 2) dodecahydro-3a,6,6,9a-tetramethyl-naphtho[2,1-b]furan; origin: Firmenich SA, Geneva, Switzerland 3) origin: Firmenich SA, Geneva, Switzerland 4) methyl dihydrojasmonate; origin: Firmenich SA, Geneva, Switzerland 5) methyl ionone; origin: Firmenich SA, Geneva, Switzerland 6) cis-7-P-menthanol; origin: Firmenich SA, Geneva, Switzerland 7) 1-(5,5-dimethyl-1-cyclohexen-1-yl)-4-penten-1-one; origin: Firmenich SA, Geneva, Switzerland 8) methyl 2,2-dimethyl-6-methylene-1-cyclohexanecarboxylate; origin: Firmenich SA, Geneva, Switzerland 19) 4-methyl-3-decen-5-ol; origin: Givaudan-Roure, Vernier, Switzerland 10) 2-tert-butyl-1-cyclohexyl acetate; origin: International Flavors & Fragrances, USA | |

The addition of 1300 parts by weight of 4,6,8a-trimethyl-3,4,4a,5,8,8a-hexahydro-1(2H)-naphthalenone or 4,8,8a-trimethyl-1,2,3,4,4a,5,8,8a-octahydro-1-naphthalenyl acetate to the above-described functional composition imparted a very elegant, as well as particularly persistent, woody-cedar note. The note imparted by 4,6,8a-trimethyl-3,4,4a,5,8,8a-hexahydro-1(2H)-napbthalenone was more elegant and warm than the one imparted by 4,8,8a-trimethyl-1,2,3,4,4a,5,8,8a-octahydro-1-naphthalenyl acetate. Furthermore, the effect imparted by the two invention's compounds was judged by the expert perfumers as being more elegant and performing, in application, than the one which was obtained by the addition of the same amount of Vertofix^{®} to the above-mentioned functional composition. The addition of the same amount of perhydro-4-ethyl-8-methyl-1-naphthalenone to the above-described functional composition imparted a very nice vetiver tonality to the new composition, while the addition of 4-ethyl-6,8-dimethyl-1,2,3,4,4a,5,8,8a-octahydro-1-naphthalenol imparted a fresh and natural citrus connotation, of the pink grapefruit type. The freshness imparted by 4-ethyl-6,8-dimethyl-1,2,3,4,4a,5,8,8a-octahydro-1-naphthalenol was also present on the dry linen, resulting thus in an exceptional performance for a compound having such fragrance notes.

## Claims

1. A perfuming composition comprising
i) as perfuming ingredient, at least one compound of formula 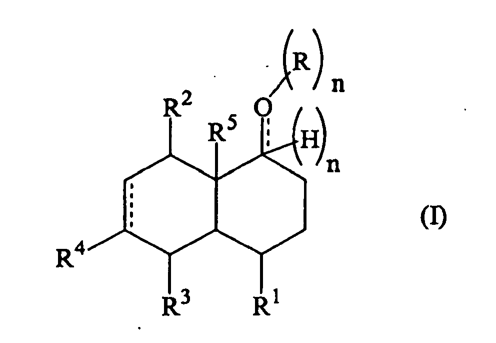 wherein the dotted lines represent a single or double bond and at least one of said dotted lines represents a double bond;
n represents simultaneously 0, in which case the oxygen atom is bounded to the cyclanic carbon atom by a double bond, or 1, in which case the oxygen atom is bounded to the cyclanic carbon atom by a single bond,
R represents a hydrogen atom or a HCO or MeCO group;
R¹ represents a linear or branched C₁-C₄ alkyl or alkenyl group;
R² represents a hydrogen atom or a methyl or ethyl group;
R³ represents, taken alone, a hydrogen atom or, taken together with R², a bridging CH₂ group;
R⁴ represents a hydrogen atom or a methyl or ethyl group; and
R⁵ represents a hydrogen atom or a linear or branched C₁-C₃ alkyl group;
in the form of any one of its stereoisomers or of a mixture thereof;
ii) at least one ingredient selected from the group consisting of a perfumery carrier and a perfumery base; and
iii) optionally at least one perfumery adjuvant.

2. A perfuming composition according to claim 1, **characterized in that** in formula (I) the dotted lines, R³ and n are as defined in claim 1;
R represents a hydrogen atom or a MeCO group;
R¹ represents a methyl, ethyl or propyl group;
each R², R⁴ and R⁵ represent a hydrogen atom or a methyl group; and
the substituents R¹, R², R³, R⁴ and R⁵ have in total 3 or 4 carbon atoms.

3. A perfuming composition according to claim 1, **characterized in that** the perfuming ingredient is a compounds of formula 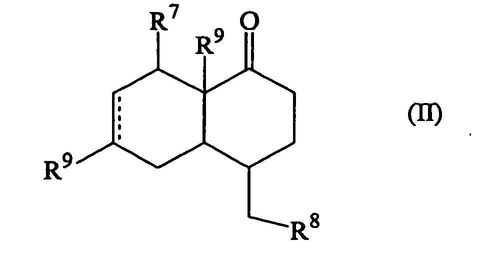 wherein the dotted lines represent a single or double bond;
R⁷ and R⁸ represent, each, a hydrogen atom or a methyl group;
R⁹ are both hydrogen atoms or are both methyl groups; and
the substituents R⁷, R⁸ and R⁹ have in total 2 or 3 carbon atoms;
or a compounds of formula 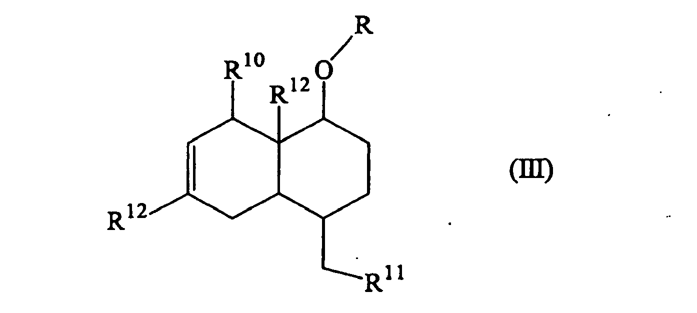 wherein R represents a hydrogen atom or a MeCO group;
R¹⁰ and R¹¹ represent, each, a hydrogen atom or a methyl group;
one R¹² is a hydrogen atom and the other is a methyl group; and
the substituents R¹⁰, R¹¹ and R¹² have in total, i.e. all together, 2 or 3 carbon atoms.

4. A perfuming composition according to claim 1, **characterized in that** the compound of formula (I) is selected from the group consisting of 4,6,8a-trimethyl-3,4,4a,5,8,8a-hexahydro-1(2H)-naphthalenone, 4,8,8a-trimethyl-1,2,3,4,4a,5,8,8a-octahydro-1-naphthalenyl acetate, perhydro-4-ethyl-8-methyl-1-naphthalenone, 4-ethyl-6,8-dimethyl-1,2,3,4,4a,5,8,8a-octahydro-1-naphthalenol, 2,6-dimethyltricyclo [6.2.1.0(2,7)]undec-9-en-3-ol, perhydro-4-ethyl-6,8a-dimethyl-1-naphthalenone, perhydro-8-methyl-4-propyl-1-naphthalenone and 4-ethyl-6,8a-dimethyl-3,4,4a,5,8,8a-hexahydro-1(2H)-naphthalenone.

5. A perfuming composition according to claim 4, **characterized in that** the compound of formula (I) is selected from the group consisting of 4,6,8a-trimethyl-3,4,4a,5,8,8a-hexahydro-1(2H)-naphthalenone, 4-ethyl-6,8-dimethyl-1,2,3,4,4a,5,8,8a-octahydro-1-naphthalenol, perhydro-8-methyl-4-propyl-1-naphthalenone and perhydro-4-ethyl-8-methyl-1-naphthalenone.

6. A compound of formula (II) or (III) as defined in claim 3, provided that 4,6,8,8a-tetramethyl-3,4,4a,5,8,8a-hexahydro-naphthalenone and 4,6,8a-trimethyl-3,4,4a,5,8,8a-hexahydro-1(2H)-naphthalenone and their isomers are excluded.

7. As a compound according to claim 6, 4-ethyl-6,8-dimethyl-1,2,3,4,4a,5,8,8a-octahydro-1-naphthalenol, perhydro-8-methyl-4-propyl-1-naphthalenone and perhydro-4-ethyl-8-methyl-1-naphthalenone.

8. A perfumed article comprising:
i) at least one compound of formula (I), as defined in any one of claims 1 to 5; and
ii) a consumer product base.

9. A perfumed article according to claim 8, in the form of a solid or liquid detergent, a fabric softener, a perfume, a cologne or after-shave lotion, a perfumed soap, a shower or bath salt, mousse, oil or gel, a hygiene product, a hair care product, a shampoo, a body-care product, a deodorant or antiperspirant, an air freshener, a cosmetic preparation, a fabric refresher, an ironing water, a paper, a wipe or a bleach.

10. A perfumed article according to claim 8, **characterized in that** the compound of formula (I) is selected from the group consisting of 4,6,8a-trimethyl-3,4,4a,5,8,8a-hexahydro-1(2H)-naphthalenone, 4-ethyl-6,8-dimethyl-1,2,3,4,4a,5,8,8a-octahydro-1-naphthalenol, perhydro-8-methyl-4-propyl-1-naphthalenone and perhydro-4-ethyl-8-methyl-1-naphthalenone.

11. Use as perfuming ingredient of a compound of formula (I) as defined in any one of claims 1 to 5.

## Patentansprüche

1. Parfümierende Zusammensetzung, aufweisend:
i) als parfümierenden Bestandteil mindestens eine Verbindung der Formel: 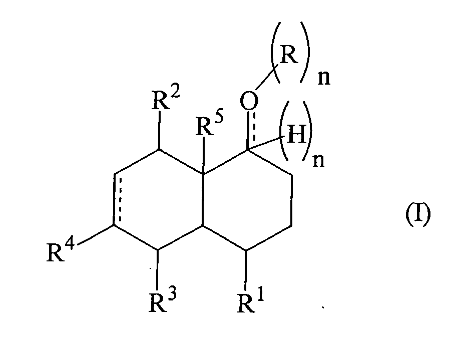 worin die gestrichelten Linien eine Einfachbindung oder eine Doppelbindung darstellen und mindestens eine der gestrichelten Linien eine Doppelbindung darstellt;
n stellt gleichzeitig Null dar, in welchem Fall das Sauerstoffatom an dem Cycloalkan-Kohlenstoffatom über eine Doppelbindung gebunden ist, oder 1 darstellt, in welchem Fall das Sauerstoffatom an dem Cycloalkan-Kohlenstoffatom über eine Einfachbindung gebunden ist,
R stellt ein Wasserstoffatom dar oder eine HCO-Gruppe oder eine MeCO-Gruppe;
R¹ stellt eine geradkettige oder verzweigte C₁-C₄-Alkyl- oder Alkenyl-Gruppe dar;
R² stellt ein Wasserstoffatom oder eine Methyl- oder Ethyl-Gruppe dar;
R³ stellt, allein genommen, ein Wasserstoffatom dar oder, zusammen genommen mit R², eine überbrückende CH₂-Gruppe;
R⁴ stellt ein Wasserstoffatom oder eine Methyl- oder Ethyl-Gruppe dar;
R⁵ stellt ein Wasserstoffatom oder eine geradkettige oder verzweigte C₁-C₃-AlkylGruppe dar;
und zwar in Form eines beliebigen seiner Stereoisomere oder eine Mischung davon;
ii) mindestens einen Bestandteil, der ausgewählt ist aus der Gruppe, bestehend aus einem Parfümträger und einer Parfümbasis; sowie
iii) wahlweise mindestens eine Parfüm-Adjuvans.

2. Parfümierende Zusammensetzung nach Anspruch 1, **dadurch gekennzeichnet, dass** in Formel (I) die gestrichelten Linien, R³ und n wie in Anspruch 1 festgelegt sind;
R ein Wasserstoffatom darstellt oder eine MeCO-Gruppe;
R¹ eine Methyl-, Ethyl- oder Propyl-Gruppe darstellt;
jedes R², R⁴ und R⁵ ein Wasserstoffatom oder eine Methyl-Gruppe darstellt und
die Substituenten R¹, R², R⁴ und R⁵ insgesamt 3 oder 4 Kohlenstoffatome haben.

3. Parfümierende Zusammensetzung nach Anspruch 1, **dadurch gekennzeichnet, dass** der parfümierende Bestandteil eine Verbindung der Formel ist: 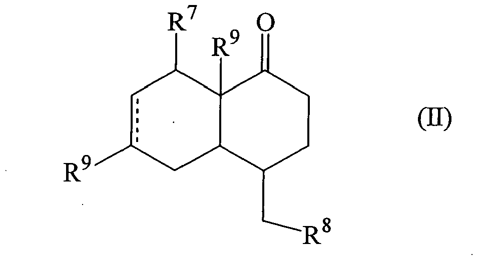 worin die gestrichelte Linien eine Einfachbindung oder eine Doppelbindung darstellen;
R⁷ und R⁸ stellen jeweils ein Wasserstoffatom dar oder eine Methyl-Gruppe;
R⁹ sind beide Wasserstoffatome oder beide Methyl-Gruppen und
die Substituenten R⁷, R⁸ und R⁹ haben insgesamt 2 oder 3 Kohlenstoffatome;
oder eine Verbindung der Formel: 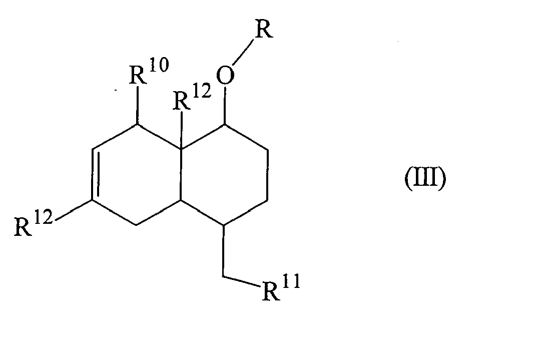 worin R ein Wasserstoffatom darstellt oder eine MeCO-Gruppe;
R¹⁰ und R¹¹ stellen jeweils ein Wasserstoffatom dar oder eine Methyl-Gruppe;
eines der R¹² ist ein Wasserstoffatom und das andere ist eine Methyl-Gruppe; und
die Substituenten R¹⁰, R¹¹ und R¹² haben insgesamt, d.h. alle zusammen, 2 oder 3 Kohlenstoffatome.

4. Parfümierende Zusammensetzung nach Anspruch 1, **dadurch gekennzeichnet, dass** die Verbindung der Formel (I) ausgewählt ist aus der Gruppe, bestehend aus: 4,6,8a-Trimethyl-3,4,4a,5,8,8a-hexahydro-1(2H)-naphthalenon, 4,8,8a-Trimethyl-1,2,3,4,4a,5,8,8a-octahydro-1-naphthalenylacetat, Perhydro-4-ethyl-8-methyl-1-naphthalenon, 4-Ethyl-6,8-dimethyl-1,2,3,4,4a,5,8,8a-octahydro-1-naphthalenol, 2,6-dimethyltricyclo[6.2.1.0(2,7)]undec-9-en-3-ol, Perhydro-4-ethyl-6,8a-dimethyl-1-naphthalenon, Perhydro-8-methyl-4-propyl-1-naphthalenon und 4-Ethyl-6,8a-dimethyl-3,4,4a,5,8,8a-hexahydro-1(2H)-naphthalenon.

5. Parfümierende Zusammensetzung nach Anspruch 4, **dadurch gekennzeichnet, dass** die Verbindung der Formel (I) ausgewählt ist aus der Gruppe, bestehend aus: 4,6,8a-Trimethyl-3,4,4a,5,8,8a-hexahydro-1(2H)-haphthalenon, 4-Ethyl-6,8-dimethyl-1,2,3,4,4a,5,8,8a-octahydro-1-naphthalenol, Perhydro-8-methyl-4-propyl-1-naphthalenon und Perhydro-4-ethyl-8-methyl-1-naphthalenon.

6. Verbindung der Formel (II) oder (III) nach Anspruch 3 unter der Voraussetzung, dass 4,6,8,8a-Tetramethyl-3,4,4a,5,8,8a-hexahydronaphthalenon and 4,6,8a-Trimethyl-3,4,4a,5,8,8a-hexahydro-1(2H)-naphthalenon und deren Isomere ausgenommen sind.

7. Verbindung nach Anspruch 6: 4-Ethyl-6,8-dimethyl-1,2,3,4,4a,5,8,8a-octahydro-1-naphthalenol, Perhydro-8-methyl-4-propyl-1-naphthalenon und Perhydro-4-ethyl-8-methyl-1-naphthalenon.

8. Parfümierter Artikel, aufweisend:
i) mindestens eine Verbindung der Formel (I), wie in einem der Ansprüche 1 bis 5 festgelegt wurde, und
ii) Verbrauchsartikel-Basis.

9. Parfümierter Artikel nach Anspruch 8 in Form eines festen oder flüssigen Detergens, eines Weichspülers, eines Parfüms, eines Köllnisch-Wassers oder After-Shave-Lotion, einer parfümierten Seife, eines Duschbads oder Badesalzes, eines Schaumproduktes, Öls oder Gels, eines Hygieneproduktes, eines Haarpflegemittels, eines Shampoos, eines Körperpflegemittels, eines Deodorans oder Antischweissmittels, eines Luftreinigers, eines kosmetischen Präparats, eines Textilerfrischers, eines Bügelwassers, eines Papiers, eines Wischmittels, eines Bleichmittels.

10. Parfümierter Artikel nach Anspruch 8, **dadurch gekennzeichnet, dass** die Verbindung der Formel (I) ausgewählt ist aus der Gruppe, bestehend aus: 4,6,8a-Trimethyl-3,4,4a,5,8,8a-hexahydro- (2H)-naphthalenon, 4-Ethyl-6,8-dimethyl-1,2,3,4,4a,5,8,8a-octahydro-1-naphthalenol, Perhydro-8-methyl-4-propyl-1-naphthalenon und Perhydro-4-ethyl-8-methyl-1-naphthalenon.

11. Verwendung eines parfümierenden Bestandteils einer Verbindung der Formel (I), wie in einem der Ansprüche 1 bis 5 festgelegt wurde.

## Revendications

1. Composition parfumante comprenant:
i) comme ingrédient parfumant, au moins un composé de la formule: 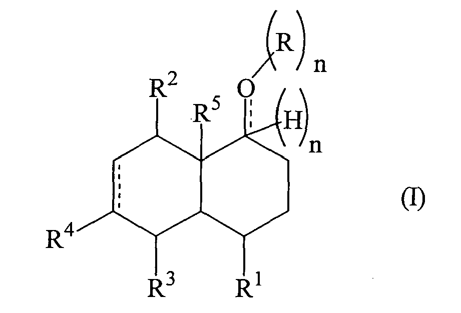 dans laquelle les lignes pointillées représentent une liaison simple ou double et au moins une desdites lignes pointillées représente une liaison double;
n représente simultanément 0, auquel cas l'atome d'oxygène est lié à l'atome de carbone cyclanique par une liaison double, ou 1, auquel cas l'atome d'oxygène est lié à l'atome de carbone cyclanique par une liaison simple,
R représente un atome d'hydrogène ou un groupe HCO ou MeCO;
R¹ représente un groupe alkyle ou alcényle C₁-C₄ linéaire ou ramifié;
R² représente un atome d'hydrogène ou un groupe méthyle ou éthyle;
R³ représente, pris seul, un atome d'hydrogène ou, pris avec R², un groupe CH₂ pontant;
R⁴ représente un atome d'hydrogène ou un groupe méthyle ou éthyle; et
R⁵ représente un atome d'hydrogène ou un groupe alkyle C₁-C₃ linéaire ou ramifié;
sous la forme de l'un quelconque de ses stéréoisomères ou d'un mélange de ceux-ci;
ii) au moins un ingrédient choisi dans le groupe constitué d'un support de parfumerie et d'une base de parfumerie; et
iii) éventuellement au moins un adjuvant de parfumerie.

2. Composition parfumante suivant la revendication 1, **caractérisée en ce que**, dans la formule (I), les lignes pointillées, R³ et n sont tels que définis dans la revendication 1;
R représente un atome d'hydrogène ou un groupe MeCO;
R¹ représente un groupe méthyle, éthyle ou propyle;
chaque R², R⁴ et R⁵ représentent un atome d'hydrogène ou un groupe méthyle; et
les substituants R¹, R², R³, R⁴ et R⁵ possèdent au total 3 ou 4 atomes de carbone.

3. Composition parfumante suivant la revendication 1, **caractérisée en ce que** l'ingrédient parfumant est un composé de la formule: 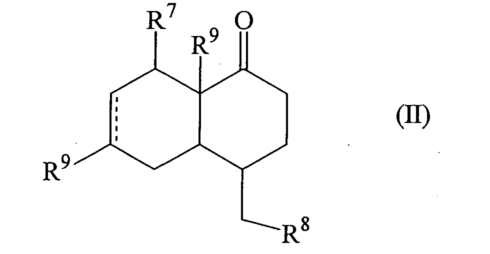 dans laquelle les lignes pointillées représentent une liaison simple ou double;
R⁷ et R⁸ représentent, chacun, un atome d'hydrogène ou un groupe méthyle;
les R⁹ sont tous les deux des atomes d'hydrogène ou sont tous les deux des groupes méthyles; et
les substituants R⁷, R⁸ et R⁹ possèdent au total 2 ou 3 atomes de carbone; ou un composé de la formule: 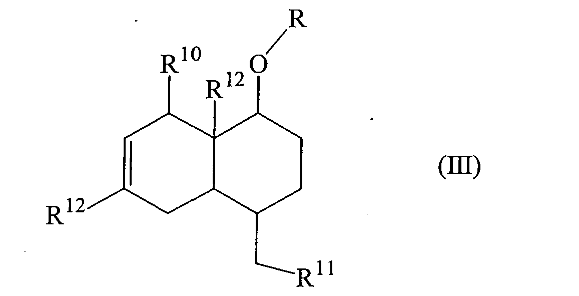 dans laquelle R représente un atome d'hydrogène ou un groupe MeCO;
R¹⁰ et R¹¹ représentent, chacun, un atome d'hydrogène ou un groupe méthyle;
un R¹² est un atome d'hydrogène et l'autre est un groupe méthyle; et
les substituants R¹⁰, R¹¹ et R¹² possèdent au total, c'est-à-dire tous ensemble, 2 ou 3 atomes de carbone.

4. Composition parfumante suivant la revendication 1, **caractérisée en ce que** le composé de la formule (I) est choisi dans le groupe constitué de 4,6,8a-triméthyl-3,4,4a,5,8,8a-hexahydro-1(2H)-naphtalénone, de 4,8,8a-triméthyl-1,2,3,4,4a,5,8,8a-octahydro-1-naphtalénylacétate, de perhydro-4-éthyl-8-méthyl-1-naphtalénone, de 4-éthyl-6,8-diméthyl-1,2,3,4,4a,5,8,8a-octahydro-1-naphtalénol, de 2,6-diméthyltricyclo [6.2.1.0(2,7)]undéc-9-én-3-ol, de perhydro-4-éthyl-6,8a-diméthyl-1-naphtalénone, de perhydro-8-méthyl-4-propyl-1-naphtalénone et de 4-éthyl-6,8a-diméthyl-3,4,4a,5,8,8a-hexahydro-1(2H)-naphtalénone.

5. Composition parfumante suivant la revendication 4, **caractérisée en ce que** le composé de la formule (I) est choisi dans le groupe constitué de 4,6,8a-triméthyl-3,4,4a,5,8,8a-hexahydro-1(2H)-naphtalénone, de 4-éthyl-6,8-diméthyl-1,2,3,4,4a,5,8,8a-octahydro-1-naphtalénol, de perhydro-8-méthyl-4-propyl-1-naphtalénone et de perhydro-4-éthyl-8-méthyl-1-naphtalénone.

6. Composé de la formule (II) ou (III) comme il est défini dans la revendication 3, à condition que la 4,6,8,8a-tétraméthyl-3,4,4a,5,8,8a-hexahydro-naphtalénone et la 4,6,8a-triméthyl-3,4,4a,5,8,8a-hexahydro-1(2H)-naphtalénone et leurs isomères soient exclus.

7. Comme composé suivant la revendication 6, le 4-éthyl-6,8-diméthyl-1,2,3,4,4a,5,8,8a-octahydro-1-naphtalénol, la perhydro-8-méthyl-4-propyl-1-naphtalénone et la perhydro-4-éthyl-8-méthyl-1-naphtalénone.

8. Article parfumé comprenant:
i) au moins un composé de la formule (I), tel que défini suivant l'une quelconque des revendications 1 à 5; et
ii) une base de produit grand-public.

9. Article parfumé suivant la revendication 8, sous la forme d'un détergent solide ou liquide, d'un assouplissant pour tissus, d'un parfum, d'une eau de Cologne ou d'une lotion après-rasage, d'un savon parfumé, d'un sel de douche ou de bain, d'une mousse, d'une huile ou d'un gel, d'un produit d'hygiène, d'un produit de soin pour les cheveux, d'un shampooing, d'un produit de soin pour le corps, d'un déodorant ou d'un anti-transpirant, d'un assainisseur d'air, d'une préparation cosmétique, d'un rafraîchissant pour tissus, d'une eau de repassage, d'un papier, d'un produit pour essuyer ou d'un agent de blanchiment.

10. Article parfumé suivant la revendication 8, **caractérisé en ce que** le composé de la formule (I) est choisi dans le groupe constitué de 4,6,8a-triméthyl-3,4,4a,5,8,8a-hexahydro-1(2H)-naphtalénone, de 4-éthyl-6,8-diméthyl-1,2,3,4,4a,5,8,8a-octahydro-1-naphtalénol, de perhydro-8-méthyl-4-propyl-1-naphtalénone et de perhydro-4-éthyl-8-méthyl-1-naphtalénone.

11. Utilisation comme ingrédient parfumant d'un composé de la formule (I) tel que défini suivant l'une quelconque des revendications 1 à 5.
